# EUROPEAN PATENT SPECIFICATION

(11) **EP 1 462 107 B1**
(45) Date of publication and mention of the grant of the patent: **24.09.2008**
(21) Application number: 03075905.4
(22) Date of filing: 28.03.2003
(51) Int. Cl.: A61K 31/565, A61K 31/57, A61P 15/18

(54) **Method of female contraception and kit for use in such method**
Weibliche Verhütungmethode und pharmazeutische Zubereitungen die für eine solche Methode geeignet sind
Procede de contraception chez des femmes et kit pharmaceutique correspondant

(43) Date of publication of application: 29.09.2004
(73) Proprietor: Pantarhei Bioscience B.V., 3701 CH Zeist (NL)
(72) Inventor: Coelingh Bennink, Herman Jan Tijmen, NL-3985 MK Werkhoven (NL); Visser, Monique, 3704 BB Zeits (NL)
(74) Representative: Jorritsma, Ruurd

(56) References cited:
- WO-A-00/07599
- WO-A-96/10991
- WO-A-99/12531
- WO-A-02/092102
- WO-A-02/094277
- WO-A-02/094281
- WO-A-03/041719
- US-A- 4 383 993

## Description

### TECHNICAL FIELD OF THE INVENTION

The present invention is concerned with a new method of contraception in mammalian females of childbearing capability. More particularly the present invention relates to such a method comprising orally administering to the female a combination of estrogen and progestogen continuously for at least 3 months.

### BACKGROUND OF THE INVENTION

Currently on the market there are a number of hormonal contraceptives for females which can be classified into two general types. The first are known as monophasic preparations. These contain a constant amount of an estrogen and a progestogen. Newer preparations known as bi- or triphasic preparations have varying levels of estrogen and progestogen; in most cases consisting of relatively constant levels of estrogen with a stepwise increase in progestogen throughout the cycle. This pattern of estrogen and progestogen administration results in a relatively dominant estrogenic formulation at the beginning of the package with increasing progestogenic activity toward the end of the package. Mono-, bi- and triphasic contraceptives are commonly referred to as combined contraceptives.

Virtually all combined contraceptives have in common that they are based on a regimen which involves an administration-free interval of about 7 days whereby withdrawal bleeding, simulating the natural menses, occurs. Thus 21 day intervals of hormone administration alternate with 7 days during which no hormones are administered.

As an alternative to the aforementioned combined contraceptive methods, the so called sequential method has been proposed. Typical of the sequential contraceptive method is that it comprises two consecutive phases, i.e. one phase during which estrogen and no progestogen is administered and another phase during which a combination of estrogen and progestogen is administered. The first sequential methods, like the aforementioned combined contraceptives, made use of an administration free interval of about 7 days. More recently, sequential methods have been proposed which do not include such an administration-free (or placebo) period, meaning that estrogen is administered throughout the full cycle and that progestogen is co-administered during only part of that cycle. WO 95/17895 (Ehrlich et al.) describes such an uninterrupted sequential method.

An uninterrupted sequential method is also described in WO 02/094218. This international patent application teaches a contraceptive method that consists of two alternating consecutive phases - an estrogenic and a progestogenic phase - said method comprising administering to a female of childbearing capability
- during the estrogenic phase one or more hormone units to provide a therapeutically effective amount of synthetic estrogen or a combination of synthetic estrogen in an amount equivalent to 3-40 µg ethinyl estradiol, and
- during the progestogenic phase one or more hormone units to provide a combination of biogenic estrogen and progestogen, said biogenic estrogen being provided in an amount equivalent to 0.5-5 mg 17β-estradiol,
wherein the progestogenic phase encompasses a period of at least 10 days and the two consecutive phases together encompass a period of 20-35 days.

Yet another alternative contraceptive method that employs continuous uninterrupted administration of progestogen and at least one estrogen is described in WO 99/12531. In contrast to the aforementioned combined and sequential contraceptive methods, no regular menses occur as the continuous administration of progestogen in the indicated amounts induces amenorrhoea. This so called continuous combined method offers the advantage that it prevents withdrawal bleedings. In addition, the method gives rise to less subjective complaints, such as the symptoms caused by hormone fluctuations, and is associated with a lower risk of VTE than the well-known combined and sequential regimens. Finally, it is believed that the avoidance of chronic fluctuations in blood serum steroid levels may have a positive impact on the occurrence of premenstrual syndrome and the risk of breast cancer.

### SUMMARY OF THE INVENTION

As mentioned above, a continuous combined contraceptive method that employs a combination of an estrogen and a progestogen is known from WO 99/12531. The present inventors have unexpectedly discovered that significantly less unscheduled bleeding and spotting is observed in such a continuous combined method if the estrogen 17β-estradiol (E2) is employed and is administered in a dosage that is significantly higher than that advocated in the PCT-application.

In addition, it was surprisingly found that the prolonged use of a combination of a relatively high dose of E2 and a progestogen very effectively suppresses endometrial thickening. This finding indicates that a continuous combined method employing a relatively high dose of E2 may be used advantageously as a contraceptive method.

It is very unexpected that, despite the use of a high dose of E2, the present method does not give rise to significant endometrial thickening since E2, like other estrogens, is usually associated with endometrial stimulation.

### DETAILED DESCRIPTION OF THE INVENTION

Accordingly, one aspect of the invention is concerned with a method of contraception in a female mammal of childbearing capability, said method comprising orally administering to the female a combination of estrogen and progestogen continuously for at least 3 months, wherein the estrogen is selected from the group consisting of 17β-estradiol, esters of 17β-estradiol and combinations thereof, said estrogen being administered in an amount equivalent to a daily oral dosage of 2.2-5 mg 17β-estradiol and said progestogen being administered in an amount equivalent to a daily oral dose of 5-50 mg dydrogesterone.

The combination of estrogen and progestogen is advantageously administered at least once daily so as to maintain a relatively constant serum concentration. Most preferably the combination is administered once daily.

The present method may suitable employ any pharmaceutically acceptable substance with sufficient progestogenic activity. The present invention encompasses the use of substances that exhibit progestogenic activity per se as well as esters of such substances. The invention also encompasses the use of progestogen metabolites that exhibit progestogenic activity. Examples of progestogens that may suitably be employed in accordance with the invention include dydrogesterone, norethisterone, levonorgestrel, norgestimate, drospirenone, 3-beta-hydroxydesogestrel, 3-keto desogestrel, 17-deacetyl norgestimate, 19-norprogesterone, acetoxypregnenolone, allylestrenol, anagestone, chlormadinone, cyproterone, demegestone, desogestrel, dienogest, dihydrodydrogesterone, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol, medrogestone, medroxyprogesterone, megestrol, melengestrol, nomegestrol, norethindrone (=norethisterone), norethynodrel, norgestrel, norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime, esters of these progestogens and combinations thereof. Preferably, the progestogen is selected from the group consisting of dydrogesterone, dihydrodydrogesterone, norethisterone, levonorgestrel, desogestrel, norgestimate, drospirenone, cyproterone, gestodene, trimegestone, progesterone, esters of these progestogens and combinations thereof. Even more preferably, the progestogen is selected from the group consisting of dydrogesteron, dihydrodydrogesterone, norethisterone, esters of these progestogens and combinations thereof. Most preferably, the progestogen is dydrogesterone, dihydrodydrogesterone or an ester thereof.

The present method may suitably employ an ester of 17β-estradiol or an ester of the progestogen. Such esters are capable of liberating 17β-estradiol or a progestogen when used in the present method, e.g. as a result of metabolic conversion. Examples of suitable esters of 17β-estradiol and progestogens include such substances wherein the hydrogen atom of at least one of the hydroxyl groups has been substituted by an acyl radical of a hydrocarbon carboxylic, sulfonic acid or sulfamic acid of 1-25 carbon atoms; tetrahydrofuranyl; tetrahydropyranal; or a straight or branched chain glycosidic residue containing 1-20 glycosidic units per residue.

Ester which can suitably be used in accordance with the invention are esters that can be obtained by reacting the hydroxyl groups of the estrogenic substances with substances that contain one or more carboxy (M⁺⁻OOC-) groups, wherein M⁺ represents a hydrogen or (akali)metal cation. Hence, in a particularly preferred embodiment, the esters are derivatives of 17β-estradiol or a progestogen, wherein the hydrogen atom of at least one of the hydroxyl groups has been substituted by -CO-R, wherein R is a hydrocarbon radical comprising from 1-25 carbon atoms. Preferably R is hydrogen, or an alkyl, alkenyl or aryl radical comprising from 1-20 carbon atoms.

An important advantage of the present method resides in the fact that it suppresses endometrial growth. Typically, in the present method, the continuous administration of the combination of estrogen and progestogen is effective in maintaining a substantially constant thin endometrium with a thickness of less than 8 mm, preferably of less than 6 mm.

The continuous administration of the combination of estrogen and progestogen is advantageously continued for a period of at least 4 months, more preferably for a period of at least 6 months. Most preferably, the present method employs continuous combined administration of the two steroids for at least 9 months.

The term "continuous" as used in here, means that the combination of estrogen and progestogen is administered at relatively regular intervals, with no (therapeutically) significant interruptions. Naturally, minor interruptions may occur that do not affect the overall effectiveness of the present method, and indeed such aberrations are encompassed by the present invention. In a preferred embodiment, and more arithmetically, the administration regimen is deemed to be continuous if the longest interval between 2 subsequent administrations is not more than 3.5 times as long as the average interval. Even more preferably said longest interval is not more than 2.5 times, most preferably not more than 1.5 times as long as the average interval.

The present method requires continuous administration of the combination of estrogen and progestogen for a period of at least 3 months. In order to minimise the risk of so called breakthrough bleeding, it can be advantageous to periodically interrupt the continuous administration for a period of 4-12 days, preferably for a period of 5-9 days. These interruptions will cause a predictable withdrawal bleeding, following which continuous administration of the combination of estrogen and progestogen can be resumed with a reduced risk of breakthrough bleeding. Preferably, such an interruption occurs after 3-12 months of continuous administration, more preferably after 3-8 months and most preferably after 3-6 months of continuous administration. In a particularly preferred embodiment, the present method employs a protocol with at least 2 interruptions following periods of continuous combined administration.

As explained before, it is a critical element of the present invention to administer a relatively high dose of estrogen. In a particularly preferred embodiment the estrogen is administered in an amount equivalent to a daily oral dosage of at least 2.4 mg 17β-estradiol, more preferably of at least 2.5 mg and most preferably of at least 2.6 mg 17β-estradiol. Preferably, the estrogen is administered in a dosage that does not exceed an amount equivalent to a daily oral dosage of 4.5 mg 17β-estradiol, more preferably it does not exceed an amount equivalent to a daily oral dosage of 4 mg 17β-estradiol and most preferably, it does not exceed an amount equivalent to a daily oral dosage of 3.5 mg 17β-estradiol.

In another preferred embodiment of the present method the progestogen is administered in an amount equivalent to a daily oral dosage of 8-30 mg dydrogesterone, more preferably in an amount equivalent to a daily oral dosage of 10-20 mg dydrogesterone. The equivalent daily oral dosages for the progestogen norethisterone acetate may be calculated by dividing the recommended dosages for dydrogesterone by 10. Similarly, equivalent dosages for other progestogens may be obtained by using conversion factors that are known in the art or that may be established by methods well known to the person skilled in the art.

The amount of estrogen administered in accordance with the present method is preferably effective to achieve a 17β-estradiol serum concentration of at least 30 pg/ml, more preferably of at least 50 pg/ml.

The present method is particularly suitable for treating humans, primates, bovines, porcines, equines, canines or felines. Most advantageously the present method is employed in the treatment of humans.

It was found that the present method is particularly advantageous in non-smoking females. Although the inventors do not wish to be bound by theory it is believed that smoking interferes with E2-metabolisation in a way that reduces the bioavailability of E2, e.g. by increasing 2-hydroxylation of E2. Thus, the present method is preferably employed to prevent conception in a non-smoking human female.

It was surprisingly found that the anti-proliferative effect of the present combination of estrogen and progestogen on the endometrium may be enhanced further by co-administration of an androgen. In addition, the continuous co-administration of an androgen in the present contraceptive regimen offers the advantage of even less vaginal breakthrough spotting and bleeding. Consequently, in another preferred embodiment of the invention, the present method comprises co-administration of an androgen.

Preferably, in the present method, androgen is co-administered in an amount effective to maintain the serum androgen concentration of the female mammal at a level equivalent to between 0.5 and 5.0, preferably between 0.7 and 4.0 and most preferably between 1.0 and 3.0 nanomoles testosterone per litre. Here the testosterone concentrations relate to the total testosterone present in the serum, i.e. including both free testosterone and bound testosterone.

The androgen used in the present method is preferably selected from the group consisting of dehydroepiandrosterone (DHEA); DHEA-sulphate; testosterone; testosterone esters such as testosterone undecanoate , testosterone propionate, testosterone phenylpropionate, testosterone isohexanoate, testosterone enantate, testosterone bucanate, testosterone decanoate, testosterone buciclate; danazol; gestrinone; methyltestosterone; mesterolon; stanozolol; androstenedione; dihydrotestosterone; androstanediol; metenolon; fluoxymesterone; oxymesterone; methandrostenolol; 7α-methyl-19-nortestosterone (MENT); precursors capable of liberating these androgens when used in the present method and mixtures thereof. Most preferably the androgen is selected from the group consisting of DHEA, danazol, gestrinone, testosterone esters, androstenedione, precursors capable of liberating these androgens when used in the present method and mixtures thereof. Preferably the testosterone esters employed in the present method comprise an acyl group which comprises at least 6, more preferably from 8-20 and preferably 9-13 carbon atoms. Most preferably the androgens used in the present method are DHEA and/or testosterone undecanoate. These androgens offer the advantage that they can effectively be used in oral dosage units.

It is noted that, for instance, DHEA, testosterone undecanoate and androstenedione are precursors of testosterone and that said precursors *per se* exhibit virtually no affinity for androgen receptors in the female body. The effectiveness of the androgens within the method of the invention is determined by their functionally active form, which may well be different from the form in which they are administered.

In a preferred embodiment the androgen is provided in an amount equivalent to a daily oral dosage of 5 to 250 mg DHEA, which is equivalent to a daily oral dosage of 1 to 50 mg testosterone undecanoate. More preferably the androgen is provided in an amount which is equivalent to a daily oral dosage of 10-120 mg DHEA. Most preferably the androgen is administered in an amount which is equivalent to a daily oral dosage of 20-60 mg DHEA.

The invention is further illustrated by means of the following examples.

### EXAMPLES

### Example 1

A clinical study was conducted in 30 young healthy female volunteers (15 smokers and 15 non-smokers) who were using a combined monophasic oral contraceptive with at least 30 microgram ethinyl estradiol at the moment of enrolment.

Immediately following 19-25 days of taking this monophasic oral contraceptive, without observing a tablet-free period, treatment with the study medication was commenced. The study medication comprised of continuously administering 3 mg 17beta-estradiol (E2) combined with 1.5 mg norethisterone acetate without pauses for 3 months. Follicular development and endometrial thickness were measured by ultrasonography once every 3 or 4 days depending on follicle growth. Vaginal spotting and bleeding was scored daily by the participants in a diary. In addition, endocrine measurements (E2 and progesterone) were performed in a subgroup of the participants (4 individuals who exhibited follicular growth, 5 randomly selected non-smokers and 5 randomly selected smokers). Specific contraceptive side-effects, mood changes and volunteer appreciation were also evaluated.

No ovulations were observed in any of the participants. In 4 of the 30 participants (2 smokers and 2 non-smokers) persistent follicles of 25-30 mm were seen. In these women, no increase in the progesterone levels was observed. No dominant follicles were seen in the other 26 women (median diameter 5 mm). Endometrial thickness did not change during treatment (median 4.5 mm before and 4.0 mm after treatment). After an initial adaptation period spotting was found to occur infrequently in 3 out of 15 non-smokers. In smokers however bleeding and spotting occurred in 3 out of 15 women and spotting only in another 5 women. In some women breast tension, bloating and acne occurred during treatment. No mood changes were registered. Of the 30 participating women, 12 would use this contraceptive when available and 12 would consider its use.

### Example 2

Example 1 is repeated with the exception that the participants continuously receive 3 mg 17beta-estradiol (E2) combined with 15 mg dydrogesterone without pauses for 4 months. Similar results are obtained as described in Example 1.

### Example 3

The study described in Example 2 is repeated with the exception that after the period of 4 months of continuous combined administration, the participants receive placebo's during 7 subsequent days, resulting in withdrawal bleeding in all subjects. Immediately following these 7 days, the participants again receive the combination of E2 and dydrogesterone uninterruptedly for another 4 months. Following this period of 4 months the women yet again receive placebo's during 7 subsequent days. Also this time, withdrawal bleeding is observed in all participants during the placebo period.

In parallel, another group of 30 women, which women were selected on the basis of the same criteria mentioned in Example 1, receives the combination of E2 and dydrogesterone continuously, i.e. without interruptions, during a period of 8 months and 14 days. It was found that the incidence of bleeding and spotting in the latter group was significantly higher than in the group that alternately received the combination of E2 and dydrogesterone for 4 months followed by an administration-free interval of 7 days.

## Claims

1. A method of contraception in a female mammal of childbearing capability, said method comprising orally administering to the female a combination of estrogen and progestogen continuously for at least 3 months, wherein the estrogen is selected from the group consisting of 17β-estradiol, esters of 17β-estradiol and combinations thereof, said estrogen being administered in an amount equivalent to a daily oral dosage of 2.2-5 mg 17β-estradiol and said progestogen being administered in an amount equivalent to a daily oral dose of 5-50 mg dydrogesterone.

2. Method according to claim 1, wherein the progestogen is selected from the group consisting of dydrogesterone, norethisterone, levonorgestrel, norgestimate, drospirenone, 3-beta-hydroxydesogestrel, 3-keto desogestrel, 17-deacetyl norgestimate, 19-norprogesterone, acetoxypregnenolone, allylestrenol, anagestone, chlormadinone, cyproterone, demegestone, desogestrel, dienogest, dihydrodydrogesterone, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol, medrogestone, medroxyprogesterone, megestrol, melengestrol, nomegestrol, norethindrone (=norethisterone), norethynodrel, norgestrel, norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime, esters of these progestogens and combinations thereof.

3. Method according to claim 2, wherein the progestogen is selected from the group consisting of dydrogesterone, dihydrodydrogesterone, norethisterone, levonorgestrel, desogestrel, norgestimate, drospirenone, cyproterone, gestodene, trimegestone, progesterone, esters of these progestogens and combinations thereof.

4. Method according to claim 3, wherein the progestogen is dydrogesterone, dihydrodydrogesterone or an ester thereof.

5. Method according to any one of the preceding claims, wherein the continuous administration of the combination of estrogen and progestogen is effective in maintaining a substantially constant endometrial thickness of less than 8 mm, preferably of less than 6 mm.

6. Method according to any one of the preceding claims, wherein the estrogen is administered in an amount equivalent to a daily oral dosage of 2.4-4 mg 17β-estradiol, preferably to a daily oral dosage of 2.5-3.5 mg 17β-estradiol.

7. Method according to any one of the preceding claims, wherein the progestogen is administered in an amount equivalent to a daily oral dosage of 8-30 mg dydrogesterone, preferably to a daily oral dosage of 10-20 mg dydrogesterone.

8. Method according to any one of the preceding claims, wherein the estrogen is administered in an amount effective to achieve a 17β-estradiol serum concentration of at least 30 pg/ml.

## Patentansprüche

1. Verfahren zur Empfängnisverhütung in einem weiblichen Säugetier mit der Fähigkeit zum Gebären von Nachkommen, wobei das Verfahren die kontinuierliche orale Verabreichung einer Kombination von Östrogen und Progestogen für wenigstens drei Monate an das Weibchen umfasst, wobei das Östrogen ausgewählt ist aus der Gruppe bestehend aus 17β-Östradiol, Ester von 17β-Östradiol und Kombinationen davon, wobei das Östrogen in einer Menge verabreicht wird, die einer täglichen oralen Dosierung von 2,2 bis 5 mg 17β-Östradiol entspricht und wobei das Progestogen in einer Menge verabreicht wird, die einer täglichen oralen Dosis von 5 bis 50 mg Dydrogesteron entspricht.

2. Verfahren nach Anspruch 1, wobei das Progestogen ausgewählt ist aus der Gruppe, bestehend aus Dydrogesteron, Norethisteron, Levonorgestrel, Norgestimat, Drospirenon, 3-beta-Hydroxydesogestrel, 3-keto Desogestrel, 17-deacetyl Norgestimat, 19-Norprogesteron, Acetoxypregnenolon, Allylestrenol, Anageston, Chlormadinon, Cyproteron, Demegeston, Desogestrel, Dienogest, Dihydrodydrogesteron, Dihydrogesteron, Dimethisteron, Ethisteron, Ethynodiol-Diacetat, Flurogeston-Acetat, Gastrinon, Gestoden, Gestrinon, Hydroxymethylprogesteron, Hydroxyprogesteron, Lynestrenol, Medrogeston, Medroxyprogesteron, Megestrol, Melengestrol, Nomegestrol, Norethindron (= Norethisteron), Norethynodrel, Norgestrel, Norgestrienon, Normethisteron, Progesteron, Quingestanol, (17alpha)-17-Hydroxy-11-Methylen-19 Norpregna-4,15-Dien-20-yn-3-on, Tibolon, Trimegeston, Algeston-Acetophenid, Nestoron, Promegeston, 17-Hydroxyprogesteron-Ester, 19-Nor-17Hydroxyprogesteron, 17Alpha-Ethinyl-Testosteron, 17alpha-Ethinyl-19-nor-Testosteron, d-17beta-Acetoxy-13beta-Ethyl-17alpha-Ethinyl-gon-4-en-3-on-Oxim, Ester dieser Progestogene und Kombinationen davon.

3. Verfahren nach Anspruch 2, wobei das Progestogen ausgewählt ist aus der Gruppe bestehend aus Dydrogesteron, Dihydrodydrogesteron, Norethisteron, Levonorgestrel, Desogestrel, Norgestimat, Drospirenon, Cyproteron, Gestoden, Trimegeston, Progesteron, Ester dieser Progesterone und Kombinationen davon.

4. Verfahren nach Anspruch 3, wobei das Progestogen Dydrogesteron, Dihydrodydrogesteron oder ein Ester davon ist.

5. Verfahren nach einem der vorhergehenden Ansprüche, wobei die kontinuierliche Verabreichung der Kombination von Östrogen und Progestogen zur Erhaltung einer im wesentlichen konstanten endometrialen Stärke von weniger als 8 mm, vorzugsweise von weniger als 6 mm wirksam ist.

6. Verfahren nach einem der vorhergehenden Ansprüche, wobei das Östrogen in einer Menge verabreicht wird, die einer täglichen oralen Dosierung von 2,4 bis 4 mg 17β-Östradiol, vorzugsweise einer täglichen oralen Dosierung von 2,5 bis 3,5 mg 17β-Östradiol entspricht.

7. Verfahren nach einem der vorhergehenden Ansprüche, wobei das Progestogen in einer Menge verabreicht wird, die einer täglichen oralen Dosierung von 8 bis 30 mg Dydrogesteron, vorzugsweise einer täglichen oralen Dosierung von 10 bis 20 mg Dydrogesteron entspricht.

8. Verfahren nach einem der vorhergehenden Ansprüche, wobei das Östrogen in einer Menge verabreicht wird, die wirksam ist, um eine 17β-Östradiol-Serum-Konzentration von wenigstens 30 µg/ml zu erreichen.

## Revendications

1. Procédé de contraception pour un mammifère femelle apte à procréer, ledit procédé comprenant l'administration orale à la femelle d'une combinaison d'oestrogène et de progestatif sans interruption pendant au moins 3 mois, dans lequel l'oestrogène est choisi dans le groupe constitué du 17β-estradiol, des esters du 17β-estradiol et des combinaisons de ceux-ci, ledit oestrogène étant administré selon une quantité équivalente à un dosage oral de 2,2 à 5 mg par jour de 17β-estradiol et ledit progestatif étant administré selon une quantité équivalente à une dose orale de 5 à 50 mg par jour de dydrogestérone.

2. Procédé selon la revendication 1, dans lequel le progestatif est choisi dans le groupe constitué de la dydrogestérone, de la noréthistérone, du lévonorgestrel, du norgestimate, de la drospirénone, du 3-β-hydroxydésogestrel, du 3-céto-désogestrel, du 17-déacétyl norgestimate, de la 19-norprogestérone, de l'acétoxyprégnènolone, de l'allylestrenol, de l'anagestone, de la chlormadinone, de la cyprotérone, de la démégestone, du désogestrel, du dienogest, de la dihydrodydrogestérone, de la dihydro gestérone, de la diméthistérone, de l'éthistérone, du diacétate d'éthynodiol, de l'acétate de flurogestone, du gastrinon, du gestodène, de la gestrinone, de l'hydroxyméthylprogestérone, de l'hydroxyprogestérone, du lynestrénol, de la médrogestone, de la médroxyprogestérone, du mégestrol, du mélengestrol, du nomégestrol, de la noréthindrone (=noréthistérone), du noréthynodrel, du norgestrel, de la norgestriénone, de la norméthistérone, de la progestérone, du quingestanol, de la (17α)-17-hydroxy-11-méthylène-19-norpregna-4,15-diène-20-yn-3-one, de la tibolone, de la trimégestone, de l'acétophénide d'algestone, de la nestorone, de la promégestone, des esters de la 17-hydroxyprogestérone, de la 19-nor-17-hydroxyprogestérone, de la 17α-éthinyl-testostérone, de la 17α-éthinyl-19-nor-testostérone, de l'oxime de d-17β-acétoxy-13β-éthyl-17a-éthinyl-gon-4-èn-3-one, des esters de ces progestatifs et des combinaisons de ceux-ci.

3. Procédé selon la revendication 2, dans lequel le progestatif est choisi dans le groupe constitué de la dydrogestérone, de la dihydrodydrogestérone, de la noréthistérone, du lévonorgestrel, du désogestrel, du norgestimate, de la drospirénone, de la cyprotérone, du gestodène, de la trimégestone, de la progestérone, des esters de ces progestatifs et des combinaisons de ceux-ci.

4. Procédé selon la revendication 3, dans lequel le progestatif est de la dydrogestérone, de la dihydrodydrogestérone ou un ester de celles-ci.

5. Procédé selon l'une quelconque des revendications précédentes, dans lequel l'administration sans interruption de la combinaison d'oestrogène et de progestatif permet de maintenir une épaisseur endométriale sensiblement constante inférieure à 8 mm, de préférence inférieure à 6 mm.

6. Procédé selon l'une quelconque des revendications précédentes, dans lequel l'oestrogène est administré selon une quantité équivalente à un dosage oral quotidien de 2,4 à 4 mg par jour de 17β-estradiol, de préférence équivalente à un dosage oral de 2,5 à 3,5 mg par jour de 17β-estradiol.

7. Procédé selon l'une quelconque des revendications précédentes, dans lequel le progestatif est administré selon une quantité équivalente à un dosage oral de 8 à 30 mg par jour de dydrogestérone, préférentiellement équivalente à un dosage oral de 10 à 20 mg par jour de dydrogestérone.

8. Procédé selon l'une quelconque des revendications précédentes, dans lequel l'oestrogène est administré selon une quantité efficace pour obtenir une concentration sérique en 17β-estradiol d'au moins 30 pg/ml.
